(19) Europäisches Patentamt
European Patent Office
Office européen des brevets

(11) **EP 3 275 396 B1**

(12) **EUROPÄISCHE PATENTSCHRIFT**

(45) Veröffentlichungstag und Bekanntmachung des Hinweises auf die Patenterteilung:
**16.06.2021 Patentblatt 2021/24**

(51) Int Cl.:
***A61C 13/00*** *(2006.01)*  ***A61C 13/09*** *(2006.01)*

(21) Anmeldenummer: **16180992.6**

(22) Anmeldetag: **25.07.2016**

(54) **VERFAHREN FÜR DIE HERSTELLUNG EINER DENTALEN RESTAURATION**

METHOD FOR THE PREPARATION OF A DENTAL RESTORATION

PROCÉDÉ DE PRODUCTION D'UNE RESTAURATION DENTAIRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**31.01.2018 Patentblatt 2018/05**

(73) Patentinhaber: **Coltène/Whaledent AG**
**9450 Altstätten (CH)**

(72) Erfinder:
• **Böhner, Ralf**
**9451 Kriessern (CH)**
• **Kopfmann, Cornelia**
**9000 St. Gallen (CH)**
• **Schlüter, Martin**
**88238 Wangen i.A. (DE)**
• **Schaufelberger, Martin**
**8872 Weesen (CH)**

(74) Vertreter: **Hepp Wenger Ryffel AG**
**Friedtalweg 5**
**9500 Wil (CH)**

(56) Entgegenhaltungen:
WO-A1-2009/070470    WO-A1-2014/153575
WO-A1-2015/051095    DE-A1- 19 654 055
JP-A- H08 112 296    US-A1- 2014 113 251

Bemerkungen:
Die Akte enthält technische Angaben, die nach dem Eingang der Anmeldung eingereicht wurden und die nicht in dieser Patentschrift enthalten sind.

**Beschreibung**

Die Erfindung betrifft ein Verfahren für die Herstellung einer dentalen Restauration.

Um zahnähnliche Restaurationen im CAD/CAM-Verfahren herzustellen werden Multicolor Blöcke verwendet. Dabei ändert sich die Farbe schichtweise oder ein andersfarbiger Kern wird verwendet. Der menschliche Zahn ist in zwei Schichten aufgebaut, dem relativ opaken und farbigen Dentin und dem relativ transluzenten und wenig farbigem Zahnschmelz. Diese Situation in einem CAD/CAM verfahren nachzubilden ist relativ schwierig und gelingt nur annähernd gut mit speziellen vorgefertigten Blöcken (bspw. RealLife Blöcke von Vita). Der vorgefertigte Block kann aber nicht jede Situation zwischen Schmelz und Dentin wiedergeben.

Mehrteilige dentale Restaurationen sind bekannt. Sie sind jedoch ästhetisch nicht ansprechend und aufwendig zu fertigen. US 4,650,418 beschreibt dentale Prothesen aus Keramik mit einer äusseren Lage, einer Zwischenlage und einer transluzenten Lage.

WO 2006/120255 A2 beschreibt Zahnersatzteile aufgebaut aus einem ersten und einem zweiten Bauteil. Die Herstellung von Zahnersatzteilen bestehend aus zwei Materialtypen ist möglich bspw.

$Al_2O_3$ oder ZrO für das erste Bauteil und Feldspat-Keramik für das zweite Bauteil. Das erste und das zweite Bauteil werden durch ein eingefärbtes Verbindungmaterial verbunden.

WO 2009/070470 offenbart ein Verfahren zur Herstellung eines Zahnersatzteils aus einem Fräsrohling, der aus mehreren Materialien besteht.

Es ist Aufgabe der Erfindung die Nachteile des Stands der Technik zu überwinden. Insbesondere ist es Aufgabe der Erfindung ein Verfahren bereitzustellen für die vereinfachte Herstellung von dentalen Restaurationen. Die Aufgaben werden durch die Merkmale des unabhängigen Anspruchs gelöst.

Die Erfindung betrifft die Herstellung einer dentalen Restauration umfassend eine äussere Komponente, insbesondere ein Schale, und ein innerer Komponente, insbesondere einen Kern. Die innere Komponente und die äussere Komponente weisen Kompositmaterial auf. Erfindungsgemäβ bestehen die erste Komponenten und die zweite Komponente aus Kompositmaterial. Die äussere Komponente wird erfindungsgemäß auf der inneren Komponente angebracht. Die innere Komponente ist zur Befestigung an eine Zahnpräparation, insbesondere einen Zahnstumpf oder Abutment, ausgestaltet. Die innere Komponente kann, insbesondere bereichsweise, eine niedrigere Transluzenz als die äussere Komponente auf weisen. Bevorzugt kann der Transluzenzunterschied im Bereich von 5 bis 15%, besonders bevorzugt im Bereich von 8 bis 15 %, liegen. Derart werden der Zahnschmelz und das Dentin des Zahns besonders gut nachgebildet. Die dentale Restauration ist so ästhetisch ansprechend.

Die Verwendung einer inneren Komponente und einer äusseren Komponente aus Kompositmaterial hat im Unterschied zu Keramik den Vorteil, dass sich das Kompositmaterial der inneren Komponente und der äusseren Komponente nicht unterschiedlich ausdehnt, sich nicht unterschiedlich verfärbt, nicht unterschiedlich Wasser aufnimmt und keinen unterschiedlichen Brechungsindex aufweist.

Die dentale Restauration aus Kompositmaterial ist homogen hinsichtlich der vorgehend genannten Eigenschaften. Die Dicke der äusseren Komponente kann bereichsweise abnehmen. Bevorzugt nimmt die Dicke der äusseren Komponente im in der Anwendungssituation cervical positionierten Bereich der dentalen Restauration ab. Derart wird der natürliche Zahn realitätsnah imitiert, um einen ästhetisch ansprechenden Übergang zwischen der Restauration und der natürlicher Zahnsubstanz zu gewährleisten.

Die Transluzenz der äusseren Komponente, insbesondere der Schale, kann im Bereich von 25 bis 40% liegen. Die Transluzenz der inneren Komponente, insbesondere des Kerns, kann im Bereich von 10 bis 28% liegen. Derart werden eine äussere Komponente und eine innere Komponenten bereitgestellt, welche die vorgenannten Transluzenzunterschiede aufweisen.

Die innere Komponente und die äussere Komponente können zumindest bereichsweise derart verklebt sein, dass ein kohäsiver Verbund aus der inneren Komponente und der äusseren Komponente ausgebildet ist. Bei Bruchversuchen mit dem Verbund entsteht ein kohäsiver Bruch, also kohäsiv im Material der inneren Komponente oder der äusseren Komponente und nicht in der Verklebung oder der Klebeflächen. Der kohäsive Bruch kann in der inneren Komponente, in der äusseren Komponente und im Kleber stattfinden. Natürlich kann der Bruch auch durch zwei der drei vorgenannten Komponenten stattfinden. Die erfindungsgemässe dentale Restauration ist also als kohäsiver Verbund ausgebildet.

Bevorzugte Klebstoffe für die Verklebung der inneren Komponente und der äusseren Komponente sind acrylhaltige Klebstoffe. Diese können eine ähnliche Zusammensetzung hinsichtlich der Kompositzusammensetzung wie die innere Komponente und die äussere Komponente aufweisen. Unterschiedliche Zusammensetzungen sind selbstverständlich auch möglich.

Ein weiterer Aspekt der Erfindung ist ein Verfahren für die Herstellung einer dentalen Restauration nach Anspruch 1. Das Verfahren umfasst den Schritt des Bereitstellens von CAD/CAM-Daten einer Zahnpräparation, insbesondere eines Zahnstumpfes, für die Herstellung einer individualisierten Restauration. Derart werden die individuellen Gegebenheiten einer Zahnpräparation im Verfahren berücksichtigt und eine individuelle Restauration ermöglicht. Das Verfahren umfasst den Schritt des Herstellens einer äusseren Komponenten, insbesondere eine Schale, aus einem ersten Kompositblock oder aus einem ersten Bereich eines mindestens zwei Bereiche umfassenden Kompositblocks und des Herstellens einer inneren Komponente, insbesondere eines Kerns, aus einem zweiten Kompositblock oder aus einem zweiten Bereich des mindestens zwei Bereiche umfassenden Kompositblocks, und das Anbringen der äusseren Komponente an der inneren Komponente; wobei die innere Komponente zur Befestigung an eine Zahnpräparation, insbesondere einen Zahnstumpf oder Abutment, ausgestaltet ist .

Der erste Kompositblock und der zweite Kompositblock oder der erste Bereich und der zweiter Bereich des zwei Bereiche umfassenden Kompostblock unterscheiden sich bevorzugt in der Zusammensetzung ihres Kompositmaterials. Derart weisen der erste und der zweite Kompositblock oder der erste Bereich und der zweite Bereich des Kompositblocks mindestens eine unterschiedliche Eigenschaft auf.

Die Transluzenz des zweiten Kompositblocks oder des zweiten Bereichs des Kompositblocks kann, insbesondere bereichsweise, niedriger sein als die Transluzenz des ersten Kompositblocks oder des ersten Bereichs des Kompositblocks. Bevorzugt ist der Transluzenzunterschied im Bereich von 5 bis 15%, besonders bevorzugt im Bereich von 8 bis 15%. Die äussere Komponente wird an der inneren Komponente angebracht. Die innere Komponente ist zur Befestigung an die Zahnpräparation, insbesondere den Zahnstumpf oder Abutment, ausgestaltet. Derart werden die äussere Komponenten und die innere Komponente einer dentalen Restauration individualisiert hergestellt; gleichzeitig werden Transluzenzen und Farbintensitäten berücksichtigt, was zu einer besonderen Ästhetik führt.

Unter einem Kompositblock wird hier und im Folgenden ein Block hergestellt aus Kompositmaterial verstanden, welcher im Wesentlichen die Form eines Quaders, eines Kubus oder einer Scheibe aufweist. Andere geometrische Formen sind prinzipiell auch möglich.

Das Kompositmaterial des oder der Kompositblöcke besteht aus einer organischen Matrix und einem Füller. Geeignete Kompositmaterialien umfassen eine organische Kunststoffmatrix (organische Phase), die mit einem anorganischen, insbesondere festen, Füllstoff versetzt ist (anorganische Phase). Mit Vorteil umfassen die organische Kunststoffmatrix wenigstens ein Methylacrylat und/oder der anorganische Füllstoff wenigstens ein Glas. Als Glas kommen bevorzugt ein Bariumglas und/oder ein Strontiumglas zum Einsatz.

Weitere bevorzugte anorganische Füllstoffe sind amorphe, z.B. kugelförmige, Materialien beispielsweise auf der Basis von Mischoxiden aus $SiO_2$, $ZrO_2$ und/oder $TiO_2$, mikrofeine Füllstoffe, wie pyrogene Kieselsäure oder Fällungskieselsäure sowie Makro-(Partikelgrösse von etwa 5 $\mu$m bis etwa 200 $\mu$m) oder Mikrofüllstoffe (Partikelgrösse von etwa 0.5 $\mu$m bis etwa 5 pm), wie Quarz (Silikate, Sande), Glaskeramiken (z.B. Barium-Aluminium-Glas) oder Glaspulver mit einer durchschnittlichen Teilchengrösse von 0.5 $\mu$m bis 5 $\mu$m sowie röntgenopake Füllstoffe, wie Ytterbiumtrifluorid. Ebenso kann der Füllstoff auch so genannte Mikrofüller-Komplexe wie z.B. Hybrid-Komposite und Nanopartikel (Nano-Hybridkomposite) umfassen. Darüber hinaus können grundsätzlich auch Glasfasern, Polyamide oder Kohlenstofffasern als Füllstoffe eingesetzt werden. Die Oberfläche des Füllstoffs ist in der Regel silanisiert, um eine Verbindung mit der organischen Matrix zu ermöglichen.

Weitere geeignete polymerisierbaren mono- oder multifunktionelle Monomere der organischen Phase sind Mono(meth)acrylate, wie Methyl-, Ethyl-, Butyl-, Benzyl-, Furfuryl- oder Phenyl(meth)acrylat, mehrfunktionelle Acrylate und Methacrylate wie zum Beispiel Bisphenol-(A)-di(meth)acrylat, Bisphenol-A-Glycidylmethacrylat (bekannt als "Bis GMA", welches ein Additionsprodukt aus Methacrylsäure und Bisphenol-A-diglycidylether ist), UDMA (ein Additionsprodukt aus 2-Hydroxyethylmethacrylat und 2,2,4-Hexamethylendiisocyanat), Di- , Tri und Tetraethyl-englykoldi(meth)acrylat (wie z.B. TEGDMA), Decandioldi(meth)acrylat, Trimethylolpropantri(meth)acrylat, Pentaerythrittetra(meth)acrylat und Butandioldi(meth)acrylat, 1,10-Decandioldi(meth)acrylat oder 1,12-Dodecandioldi(meth)acrylat. Ebenso sind organische Phasen umfassend methacrylatmodifizierte Polysiloxane bekannt.

Das Kompositmaterial der Kompositblöcke kann > 40 Gew.-% eines Füllers, im Sinne eines Füllstoffes oder einer Mischung von Füllstoffen, aufweisen. Dabei ist insbesondere eine relative Zusammensetzung vorgesehen, welche eine Einzelkomponente oder Zusammensetzungen aus Einzelkomponenten darstellt. Der Füller beziehungsweise die Füller können singulär, im Sinne von isolierten Füller sein, aber auch agglomeriert oder als Cluster vorliegen. Unter Agglomeraten werden allgemein mehr oder weniger verfestigte Anhäufungen von vorher losen Bestandteilen zu einem festen Verbund verstanden. In der Verfahrenstechnik werden Agglomerate beispielsweise durch Granulieren, Flockieren oder Sintern erhalten. Die Cluster können dabei mechanisch -oder chemisch verbunden sein. Die vorgenannten > 40 Gew.-% des Füllers können 0 bis 100 Gew.-% Dentalglas mit einer durchschnittlichen Partikelgrösse von 0,1 bis 5 $\mu$m (beispielsweise erhältlich von Schott), 0 bis 100 Gew.-% Splitter aus organischer Matrix und einem organischen Füller mit einer durchschnittlichen Partikelgrösse von 1 bis 30 $\mu$m aufweisen. Dabei entsprechen die Splitter einem gemahlenen Kompositmaterial. Des Weiteren umfasst die Zusammensetzung 0 bis 100 Gew.-% im Sol-Gel-Verfahren hergestellte anorganische Füller mit einer durchschnittlichen Partikelgrösse von 0,1 bis 5 $\mu$m sowie 0 bis 100 Gew.-% einer insbesondere pyrogenen Kieselsäure mit einer durchschnittlichen Partikelgrösse von 0,002 bis 0,25 pm. Auf diese Weise werden Kompositblöcke bereitgestellt, welche den Anforderungen im Dentalbereich, beispielsweise hinsichtlich Abrasionsfähigkeit, Kompressionshärte und Langlebigkeit, gerecht werden.

Die Abmessungen der Kompositblöcke können eine Breite im Bereich von 0,5 bis 5 cm, insbesondere 1 bis 3 cm, und/oder eine Grundfläche im Bereich von 0,25 bis 25 cm$^2$, insbesondere 1 bis 9 cm$^2$, aufweisen. Ebenso sind Abmessungen von 12 cm x 3 cm, insbesondere 10 cm x 2,5 cm, vorsehbar. Auf diese Weise können Komponenten der dentalen Restaurationen unterschiedlichster Grössen aus dem Kompositblock hergestellt werden.

Die Transluzenz des ersten Kompositblocks oder des ersten Bereichs des Kompositblocks kann im Bereich von 25 bis 40% liegen. Die Transluzenz des zweiten Kompositblocks oder des zweiten Bereichs des Kompositblocks kann im Bereich von 10 bis 28% liegen. Derart werden eine äussere Komponente und eine innere Komponenten bereitgestellt, welch die vorgenannten Transluzenzunterschiede aufweisen.

Die innere Komponente und die äussere Komponente können zumindest bereichsweise derart verklebt werden, dass ein kohäsiver Verbund ausgebildet wird; dies insbesondere bereichsweise im Kontaktbereich, welcher zwischen der inneren Komponente und der äusseren Komponente ausgebildet wird. Alternativ können die innere Komponente und die äussere Komponenten im gesamten Kontaktbereich zwischen der inneren Komponente und der äusseren Komponente verklebt werden. Alternativ kann der erste Bereich oder der zweite Bereich des Kompositblocks zumindest bereichsweise oder im gesamten Kontaktbereich derart verklebt sein, dass ein kohäsiver Verbund ausgebildet wird. Derart wird ein kohäsiver Verbund erzielt, welcher die bereits vorgängig erläutert Vorteile hinsichtlich des Bruchverhaltens aufweist. Besonders bevorzugte Klebstoffe sind die vorgängig erwähnten acrylhaltigen Klebstoffe.

TRANSLUZENZMESSUNG

Die Messung erfolgte an einem UV/Vis Spectrophotometers (Specord 210 von AnalytikJena) nach Messanweisung angepasst von AnalytikJena, Farbemessung und Bestimmung der Transluzenz von Dentin, Referenz-Nr.: 02/2010.

Mit der Integrationskugel sind Transmissions- und Remissionsmessungen streuender fester und flüssiger Proben sowie von Pulverproben möglich. Für die Bestimmung der Transluzenz von Dentalmaterialien werden Reflexionsspektren im VIS-Bereich aufgenommen, welche mittels Farbsoftware ausgewertet werden.

Als Referenz wurde der Spektraloneinsatz der Kugel verwendet. Für die Bestimmung der Transluzenz wurde ein Plättchen (1mm) verwendet, welches jeweils ohne Hintergrund (Schwarz-Standard) und mit einem Weiss-Standard als Hintergrund vermessen wurde. Der Weiss-Standard wurde hierbei auch als Referenz verwendet.

Die Aufnahme der Remissionsspektren erfolgte unter Einstellung der Lichtart D65 und einem Observer von 10°. Es wurden folgende Parametereinstellungen vorgenommen:

| Gerät | Specord 210 |
|---|---|
| Zubehör | Integrationskugel |
| Anzeige | Transmission |
| Korrektur | Referenz |
| Spalt | 4 nm |
| Lampenwechsel | 320 nm |
| Messmodus | Schrittbetrieb |
| Bereich [nm] | 380 - 780 nm |
| Schrittweite [nm] | 1 nm |
| Integrationszeit [s] | 0.2 s |

Zur Überprüfung der Richtigkeit der Methode wurden zusätzlich drei zertifizierte Farbstandards gemessen.

Die Transluzenz T (in %) wurde anschliessend aus folgender Formel berechnet:

$$\%T = ((L_{weiss} - L_{schwarz}) \,/\, L_{weiss}) \times 100\%$$

Nachfolgend wird die Erfindung anhand von Figuren exemplarischer Ausführungsbeispiele näher erläutert.

Es zeigen:

Figur 1:   Eine schematische Schnittdarstellung einer Ausführung der Restauration;
Figur 2:   Ein Flussdiagramm einer Ausführung des erfindungsgemässen Verfahrens;
Figur 3:   Eine schematische Darstellung eines nichtindungsgemäßen Kompositblocks aufweisend zwei Bereiche;

Figur 4:     Eine schematische Darstellung eines nichterfindungsgemäßen Kompositblocks aufweisend drei Bereiche.

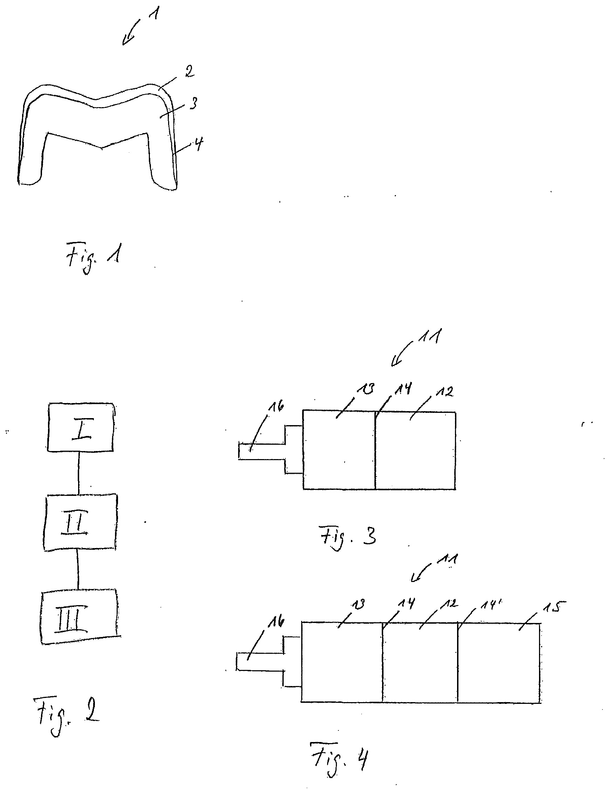

Figur 1 zeigt eine dentale Restauration 1 umfassend eine Schale 2 und einen Kern 3. Die Schale 2 und der Kern 3 sind aus Kompositmaterial gefertigt. Die Schale 2 ist mit dem Kern 3 im Kontaktbereich 4 zwischen der Schale 2 und dem Kern 3 verklebt. Als Klebstoff wird ein acrylhaltiger Klebstoff verwendet, welcher eine ähnliche Kompositzusammensetzung wie die Schale 2 und der Kern 3 aufweist. Die Verklebung im Kontaktbereich 4 führt zu einem Verbund mit kohäsiven Brucheigenschaften. Bei Bruchtests bricht der Verbund nicht an der Klebestelle oder Klebefläche sondern im Material der Schale 2 oder des Kerns 3. Der Verbund aus Schale 2 und Kern 3 bildet einen kohäsiven Verbund. Der Kern 3 ist derart ausgestaltet, dass er an eine Zahnpräparation befestigt werden kann. Der Kern 3 weist eine niedrigere Transluzenz auf als die Schale 2. Der Transluzenzunterschied zwischen Schale 2 und Kern 3 liegt im Bereich von 5 bis 15%.

Beispielhaft weist die Schale 2 eine Transluzenz von 30% und der Kern eine Transluzenz von 20% auf.

Die Dicke der Schale 2 nimmt in den in der Anwendungssituation cervical positionierten Bereichen der dentalen Restauration 1 ab. Die Schale 2 ist also auslaufend ausgestaltet. Derart wird der natürliche Zahn realitätsnah imitiert, um einen ästhetisch ansprechenden Übergang zwischen der Restauration und der natürlicher Zahnsubstanz zu gewährleisten.

Figur 2 zeigt ein Flussdiagramm einer Ausführung des erfindungsgemässen Verfahrens. Das Verfahren betrifft die Herstellung einer dentalen Restauration. Das Verfahren umfasst den Schritt I des Bereitstellens von CAD/CAM-Daten einer Zahnpräparation für die Herstellung einer individualisierten Restauration. Derart werden die individuellen Gegebenheiten einer Zahnpräparation im Verfahren berücksichtigt und eine individuelle Restauration ermöglicht.

Das Verfahren umfasst den Schritt II, wobei eine Schale (vgl. Schale 2 in Fig. 1) aus einem ersten Kompositblock und eine Kern (vgl. Kern 3 in Fig. 1) aus einem zweiten Kompositblock hergestellt wird. Dies erfolgt in einem CAD/CAM-Verfahren. Die Transluzenz des zweiten Kompositblocks für die Herstellung des Kerns ist niedriger als die Transluzenz des ersten Kompositblocks für die Herstellung der Schale. Der Transluzenzunterschied zwischen Schale und Kern ist im Bereich von 5 bis 15%. Der Kern ist zur Befestigung an die Zahnpräparation ausgestaltet.

Das Verfahren umfasst des Weiteren den Schritt III, wobei die Schale und der Kern verklebt werden. Die Verklebung führt zu den vorteilhaften Eigenschaften der Restauration wie für Figur 1 hinsichtlich eines kohäsiven Verbunds erläutert. Derart werden des Weiteren die Schale und der Kern einer dentalen Restauration individualisiert hergestellt; gleichzeitig werden Transluzenzen und Farbintensitäten berücksichtigt, was zu einer besonderen Ästhetik führt.

Figur 3 zeigt einen nicht-erfindungsgemäßen Kompositblock 11 aufweisend einen ersten Bereich 12 und einen zweiten Bereiche 13. Der erste Bereich 12 und der zweite Bereiche 13 sind im Kontaktbereich 14 miteinander verbunden. Die Bereiche 12 und 13 können auch umgekehrt angeordnet sein.

Das Kompositmaterial des ersten Bereichs 12 unterscheidet sich vom Kompositmaterial des zweiten Bereichs 13 in mindestens einer Eigenschaft. Das Kompositmaterial des ersten Bereichs 12 weist hier eine niedrigere Transluzenz als das Kompositmaterial des Bereichs 13 auf. Aus dem Bereich 13 kann beispielsweise zunächst ein Kern einer individuellen dentalen Restauration in einem CAD/CAM-Verfahren hergestellt werden. Hierzu wird der Kompositblock 11 mittels einer Halterung 16 in eine entsprechende Vorrichtung eingespannt. Ohne ein weiteres Umspannen kann aus dem ersten Bereich 12 eine Schale der individuellen dentalen Restauration hergestellt werden. Die Verwendung des Kompositblocks 11 in einem CAD/CAM-Verfahren zur Herstellung einer individuellen dentalen Restauration optimiert das Herstellungsverfahren, da der Kompositblock 11 die Materialien für eine Schale und einen Kern bereitstellt.

Figur 4 zeigt einen nicht-erfindungsgemäßen Kompositblock 11. Die Erläuterungen zu Figur 4 gelten gleichermassen. Der Kompositblock 11 der Figur 4 weist einen dritten Bereich 15 auf, welcher mit dem ersten Bereich 12 über einen Kontaktberiech 14' in Verbindung steht. Derart stellt der Kompositblock 11 ein drittes Kompositmaterial zur Verfügung, welches sich mindestens in einer Eigenschaft von den Kompositmaterialien der Bereiche 12 und 13 unterscheidet. Derart kann eine weitere Komponente aus dem Kompositblock 11 in einem CAD/CAM-Verfahren gefertigt werden, ohne dass ein Umspannen, sprich ein Einspannen eines weiteren Kompositblocks, notwendig ist.

## Patentansprüche

1. Verfahren für die Herstellung einer dentalen Restauration (1) umfassend eine individualisiert hergestellte, äussere Komponente (2) und eine individualisiert hergestellte, innere Komponente (3),

   - wobei die innere Komponente (3) und die äussere Komponente (2) Kompositmaterial aufweisen;
   - wobei die äussere Komponente (2) auf der inneren Komponente (3) anbringbar ist,
   - wobei die innere Komponente (3) zur Befestigung an eine Zahnpräparation, insbesondere einen Zahnstumpf oder Abutment, ausgestaltet ist,

umfassend die Schritte

- Bereitstellen von CAD/CAM-Daten einer Zahnpräparation für die Herstellung einer individualisierten Restauration;
- Individualiertes Herstellen einer äusseren Komponente (2) aus einem ersten Kompositblock oder aus einem ersten Bereich (12) eines mindestens zwei Bereiche umfassenden Kompositblock unter Berücksichtigung der individuellen Gegebenheiten einer Zahnpräperation; und individualisiertes Herstellen einer inneren Komponente (3) aus einem zweiten Kompositblock oder aus einem zweiten Bereich (13) des mindestens zwei Bereiche umfassenden Kompositblocks unter Berücksichtigung der individuellen Gegebenheiten einer Zahnpräperation,
- Anbringen der äusseren Komponente (2) an der inneren Komponente (3);

wobei die innere Komponente (3) zur Befestigung an eine Zahnpräparation, insbesondere einen Zahnstumpf oder Abutment, ausgestaltet ist.

2. Verfahren nach Anspruch 1, wobei die Transluzenz des zweiten Kompositblocks oder des zweiten Bereichs (13) des Kompositblocks, insbesondere bereichsweise, niedriger ist als die Transluzenz des ersten Kompositblocks oder des ersten Bereichs (13) des Kompositblocks und/oder der Transluzenzunterschied des ersten und des zweiten Kompositblocks oder des ersten Bereichs und des zweiten Bereich des Kompositblocks im Bereich von 5 bis 15%, bevorzugt im Bereich von 8 bis 15%, liegt.

3. Verfahren nach Anspruch 1 oder 2, wobei die Transluzenz des ersten Kompositblocks oder des ersten Bereichs (12) des Kompositblocks im Bereich von 25 bis 40% liegt und/oder die Transluzenz des zweiten Kompositblocks oder des zweiten Bereichs (13) des Kompositblocks im Bereich von 10 bis 28% liegt.

4. Verfahren nach einem der Ansprüche 1 bis 3, wobei die innere Komponente (3) und die äussere Komponente (2) zumindest bereichsweise derart verklebt werden oder der erste Bereich (12) und der zweiter Bereich (13) des Kompositblocks derart verklebt sind, dass ein kohäsiver Verbund ausgebildet wird.

**Claims**

1. Method for the manufacture of a dental restoration (1) comprising an individually manufactured outer component (2) and an individually manufactured inner component (3),

- wherein the inner component (3) and the outer component (2) comprise composite material;
- the outer component (2) being attachable to the inner component (3),
- wherein the inner component (3) is designed for attachment to a tooth preparation, in particular a tooth stump or abutment,

comprising the steps

- providing CAD/CAM data of a tooth preparation for the production of an individualised restoration;
- individualised production of an outer component (2) from a first composite block or from a first region (12) of a composite block comprising at least two regions, taking into account the individual circumstances of a tooth preparation; and individualised production of an inner component (3) from a second composite block or from a second region (13) of the composite block comprising at least two regions, taking into account the individual circumstances of a tooth preparation,
- attaching the outer component (2) to the inner component (3);

wherein the inner component (3) is designed for attachment to a tooth preparation, in particular a tooth stump or abutment.

2. Method according to claim 1, wherein the translucency of the second composite block or of the second region (13) of the composite block, in particular region-wise, is lower than the translucency of the first composite block or of the first region (13) of the composite block and/or the difference in translucency of the first and the second composite block or of the first region and the second region of the composite block is in the range of 5 to 15%, preferably in the range of 8 to 15%.

**3.** Method according to claim 1 or 2, wherein the translucency of the first composite block or the first region (12) of the composite block is in the range of from 25 to 40% and/or the translucency of the second composite block or the second region (13) of the composite block is in the range of from 10 to 28%.

**4.** Method according to any one of claims 1 to 3, wherein the inner component (3) and the outer component (2) are at least partially bonded or the first region (12) and the second region (13) of the composite block are bonded such that a cohesive bond is formed.

**Revendications**

**1.** Un procédé de fabrication d'une restauration dentaire (1) comprenant un composant extérieur (2) fabriqué individuellement et un composant intérieur (3) fabriqué individuellement,

- dans laquelle le composant interne (3) et le composant externe (2) sont constitués d'un matériau composite;
- ledit composant extérieur (2) pouvant être fixé audit composant intérieur (3),
- dans laquelle le composant interne (3) est conçu pour être fixé à une préparation dentaire, en particulier à un moignon de dent ou à un pilier,

comprenant les étapes

- fournir les données CAO/FAO d'une préparation dentaire pour la production d'une restauration individualisée;
- fabrication individualisée d'un composant extérieur (2) à partir d'un premier bloc composite ou d'une première région (12) d'un bloc composite comprenant au moins deux régions, en tenant compte des conditions individuelles d'une préparation dentaire ; et fabrication individualisée d'un composant intérieur (3) à partir d'un deuxième bloc composite ou d'une deuxième région (13) du bloc composite comprenant au moins deux régions, en tenant compte des conditions individuelles d'une préparation dentaire,
- la fixation de l'élément extérieur (2) à l'élément intérieur (3);

dans laquelle le composant interne (3) est conçu pour être fixé à une préparation dentaire, en particulier à un moignon de dent ou à un pilier.

**2.** Procédé selon la revendication 1, dans lequel la translucidité du deuxième bloc composite ou de la deuxième région (13) du bloc composite, en particulier par région, est inférieure à la translucidité du premier bloc composite ou de la première région (13) du bloc composite et/ou la différence de translucidité des premier et deuxième blocs composites ou de la première région et de la deuxième région du bloc composite est comprise entre 5 et 15 %, de préférence entre 8 et 15 %.

**3.** Procédé selon la revendication 1 ou 2, dans lequel la translucidité du premier bloc composite ou de la première région (12) du bloc composite se situe dans la plage de 25 à 40 % et/ou la translucidité du deuxième bloc composite ou de la deuxième région (13) du bloc composite se situe dans la plage de 10 à 28 %.

**4.** Procédé selon l'une quelconque des revendications 1 à 3, dans lequel le composant interne (3) et le composant externe (2) sont au moins partiellement collés ou la première région (12) et la deuxième région (13) du bloc composite sont collées de telle sorte qu'une liaison cohésive est formée.

Fig. 1

Fig. 2

Fig. 3

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4650418 A
- WO 2006120255 A2
- WO 2009070470 A